Europäisches Patentamt

European Patent Office

Office européen des brevets

(11) Publication number : **0 472 428 A2**

# (12) EUROPEAN PATENT APPLICATION

(21) Application number : **91307728.5**

(51) Int. Cl.⁵ : **A61K 9/68,** A23G 3/30

(22) Date of filing : **22.08.91**

(30) Priority : **23.08.90 US 571497**

(43) Date of publication of application :
**26.02.92 Bulletin 92/09**

(84) Designated Contracting States :
**DE DK ES FR GB NL**

(71) Applicant : **WM. WRIGLEY JR. COMPANY**
**410 North Michigan Avenue**
**Chicago Illinois 60611 (US)**

(72) Inventor : **Vincent, C. Bonica**
**3 Dorian Road**
**Metuchen, New Jersey 08840 (US)**
Inventor : **Foster, John W.**
**125 Florence Avenue**
**Piscataway, New Jersey 08854 (US)**
Inventor : **Synosky, Steven P.**
**56 Brandywine Rise**
**Greenbrook, New Jersey 08812 (US)**

(74) Representative : **Allard, Susan Joyce et al**
**BOULT, WADE & TENNANT, 27 Furnival Street**
**London EC4A 1PQ (GB)**

(54) Low moisture chewing gum.

(57)    A low-moisture gum includes about 30 to about 90 percent of a gum base with a softening point from about 70°C to about 100°C ; from about 0.1 to about 20 percent of a water-insoluble plasticizer ; from about 0 to about 65 percent solid bulk sweetener ; less than about 0.5 percent water ; and is substantially free of humectant.

EP 0 472 428 A2

This invention relates to a novel low-moisture chewing gum. Low-moisture gums are useful for improving the stability and the physical and chemical characteristics of certain ingredients, and for providing long-term shelf life of the product.

Low-moisture chewing gums can also be useful vehicles for the delivery of compounds such as pharmaceutical agents and artificial sweeteners. Chewing gums offer advantages to oral delivery, depending on factors such as those that alter their rate of release from the gum (e.g. the substance's degree of water-solubility) which may be particularly advantageous in oral administration of e.g. medicants.

Since moisture can hydrolyze some artificial sweeteners and pharmaceuticals and can cause instability of the gum due to moisture loss, low-moisture gums can provide long-term stability of these ingredients and of the gum product itself.

While some low-moisture gums have been proposed in the past using low softening point ingredients to make up for the lack of moisture, low softening point ingredients have a potential problem. Gums with low softening point ingredients have to be formulated with a high percentage of gum base (e.g., 90 percent or more) because other ingredients such as flavoring oils can make the gum too soft. Thus, the amount of bulk sweetener or other flavorings can be too low and may not adequately mask any objectionable taste the pharmaceutical may have.

Other low-moisture gums use humectants such as glycerine to plasticize the gums. During storage of the gum, humectants absorb atmospheric water, which can cause the gum to stick to the packaging material, can cause instability such as an unsatisfactory pH environment (due to the increased moisture content), and can cause the gum to have a soupy consistency. The increase in moisture during storage also defeats many of the other advantages low-moisture gums offer.

This invention provides a low-moisture gum that is substantially free of humectants, is shelf-stable, and offers an environment that improves the stability of ingredients such as pharmaceuticals and artificial sweeteners. Furthermore, the gum employs a base that can accommodate fairly high levels of sweetener and other flavorings that can mask the flavor of many pharmaceuticals. This invention also involves a method of pharmaceutical administration using the gum.

The gum composition of this invention employs in combination from about 30 percent to about 90 percent by weight of a gum base with a softening point from about 70°C to about 100°C; from about 0.1 percent to about 20 percent by weight of a water-insoluble plasticizer; from about 0 percent to about 65 percent of a solid bulk sweetener; less than about 0.5 percent water; and substantially free of humectant.

As indicated previously, the gum composition of this invention includes from about 30 to 90 percent by weight of a gum base with a softening point from about 70°C to about 100°C, preferably between 70 percent and 80 percent by weight. The softening point of the base is determined by using ASTM method E28-58T modified whereby the gum base sample is softened in an oven and molded into a shouldered brass ring and trimmed. The sample is placed in a water and glycerin bath and adjusted to a temperature rise of 1°C ±0.25°C per minute.

The typical gum base composition used for this invention is comprised of elastomers, elastomer solvents, fillers, and softeners. The elastomers may be synthetic, natural and/or combinations thereof. The synthetic elastomers can include but are not limited to, isobutylene-isoprene copolymers, styrene-butadiene copolymers, polyisobutylene, polyvinyl acetate, vinyl laurate copolymers, and ethylene vinyl acetate copolymers.

The natural elastomers may include chicle, jelutong, gutta percha, natural rubber, leche caspi and other natural elastomers commonly used in gum bases. The elastomer portion of this base composition ranges from about 5 percent to about 75 percent by weight of the gum base, preferably from about 5 percent to about 55 percent.

The elastomer solvents used in the base may include glycerol esters of partially hydrogenated wood or gum rosin, glycerol ester of gum, wood or tall oil rosin, glycerol ester of polymerized rosin, glycerol ester of partially dimerized rosin, polyterpene resins and mixtures thereof. The elastomer solvents may be used in an amount ranging from about 10 percent to about 50 percent by weight of gum base and preferably from about 20 percent to about 45 percent.

The gum base composition can also include fillers such as talc, calcium carbonate, magnesium silicate or combinations thereof. The amount of filler used in the base of this invention ranges preferably from about 0 percent by weight of the base to about 50 percent, preferably from 20 percent to 50 percent by weight.

The base composition may also include softeners and texture modifiers such as microcrystalline, paraffin and synthetic waxes, lecithin, glycerol monostearate, triacetin, hydrogenated and partially hydrogenated vegetable oils. These additional ingredients may range in amount from about 5 percent by weight of the base to about 50 percent and preferably from about 7 percent to about 40 percent.

The water-insoluble plasticizers include but are not limited to lecithin, acetylated or distilled monoglycerides, paraffin waxes, glycerol triacetate, hydrogenated and partially hydrogenated vegetable oils, glycerol monosearate, vegetable oil (not hyrdrogenated) and animal fats, glycerol monolaurate, coconut oil and mixtures thereof. The water-insoluble plasticizer content ranges between about 0.1 percent to about 20 percent, preferably from about 0.5 percent to about 10 percent, by weight of the gum composition.

The solid bulk sweetener makes up from about 0 to about 65 percent preferably 15 to 25 percent of the gum by weight. Such sweeteners include sugars and sugar alcohols such as sucrose, dextrose, sorbitol, maltitol, mannitol, xylitol, palatinit, polydextrose and the like.

Artificial or non-nutritive sweeteners may also be added to the gum of this invention. Such sweeteners include saccharin, aspartame, acesulfame-k and the like. The amount of sweetener used is a matter of preference.

Flavorings are also preferably included in the gum. Flavorings are often oil-soluble liquids but some are spray-dried. Flavorings may be natural or artificial. Preferred flavors are peppermint, spearmint, cinnamon and fruit flavors. The amount of flavor used depends on the type of base used; the higher softening point bases, for example, can accommodate a greater amount of oil-soluble flavoring without the flavor oils unduly plasticizing the gum. The amount of flavor used also depends on the type of flavoring; some flavors have fairly high affinity toward the elastomers in the base, the higher the affinity, the more flavor that must be used to obtain the same level of flavor perception. An advantage of this invention is that high levels of flavoring can be used to achieve a prolonged perception of flavor, yet the gum will not be unduly softened by the flavoring. Typically, the amount of flavor will be no more than about 3 percent by weight of the gum.

The flavoring can be encapsulated by spray-drying on a poorly soluble substrate such as maltodextrin or starches. These encapsulated flavors are readily available in the flavor trade.

The flavoring can also include a food acid such as citric, malic, ascorbic, odipic, tartaric, or buffering agent such as sodium citrate or potassium phosphate.

Food acids or buffering agents can be added to alter the pH of the gum. This can be important when a particular additive requires a specific pH to prolong its shelf life or improve its efficacy during chewing.

As described above, the gum composition of this invention should be substantially free of humectants. Humectants are substances that are water soluble and have an affinity for water. They tend to absorb water from the atmosphere. If excessive levels of humectants are used, the gum can absorb water from the atmosphere in amounts that can adversely affect the chewing consistency and stability of the gum and/or pharmaceutical. Humectants such as glycerin are also used in low-moisture gums to plasticize them. Gum compositions of the invention by contrast are substantially free of humectant which means their moisture levels can stay below 0.5 percent over extended periods of shelf storage yet still be soft and flexible.

The pharmaceuticals that can be delivered in a physiologically effective amount in the gum of this invention include stimulants (e.g., nicotine, caffeine), antibiotics, water-soluble vitamins (e.g., Vitamin B, C), and coenzymes (e.g. biotin, folic acid), minerals (e.g. $Ca^{2+}$), salts (e.g. $Na^+$, $K^+$, $Cl^-$), and dentifrices (e.g. fluoride). By "physiologically effective amount" is meant an amount that achieves the desired physiological response or effect intended for the pharmaceutical in question. The dose/response relationship is known for many of the classes of pharmaceuticals above.

When the ingredients described above are blended together, and the melting point is controlled as described, the gum composition can accommodate fairly high amounts of sweetener and/or flavoring. Such high sweetener and flavoring levels in a low-moisture gum masks the flavor of a pharmaceutical well and is good tasting and pleasant. Yet the gum composition is a media in which pharmaceuticals are stable.

The gum composition can be encapsulated with a coating of sucrose, dextrose or sugar alcohols such as sorbitol, mannitol or xylitol. They can even be coated with polymeric films used in the pharmaceutical industry, such as ethyl cellulose or methyl cellulose.

The following examples illustrate gum compositions of this invention.

## EXAMPLE I

### Gum Bases

A gum base of any of the formulae A-E of Table I is prepared by adding a portion of the elastomer, elastomer solvent and filler to a heated sigma blade mixer with a front to rear speed ratio of typically 2:1. The initial amounts of ingredients are determined by the working capacity of the mixing kettle in order to attain a proper consistency. After the initial ingredients have massed homogeneously, the balance of the elastomer solvent, filler, softeners, etc. is added in a sequential manner until a completely homogeneous molten mass is attained. This can usually be achieved in one to three hours, depending on the formulation. The final mass temperature can be between 70°C and 130°C and preferably between 100°C and 120°C. The completed molten mass is emptied from the mixing kettle into coated or lined pans, extruded or cast into any desirable shape and allowed to cool and solidify.

TABLE I

| | A | B | C | D | E |
|---|---|---|---|---|---|
| **Natural Elastomer** | | | | | |
| Jelutong | 13 | – | – | 4 | – |
| **Synthetic Elastomers** | | | | | |
| Isobutylene-isoprene copolymer | 7 | – | – | 8 | 9 |
| Styrene-butadiene copolymer | – | 9 | – | – | – |
| Polyisobutylene | – | – | 14 | 3 | – |
| Polyvinyl acetate | 12 | – | 19 | 4 | – |
| **Elastomer Solvents** | | | | | |
| Terpene resin | 9 | – | – | 6 | – |
| Rosin ester | 11 | 37 | 10 | 18 | 37 |
| **Fillers** | | | | | |
| Calcium carbonate | – | 45 | 39 | 18 | – |
| Talc | 15 | – | – | – | 45 |
| **Softeners** | | | | | |
| Microcrystalline wax | 27 | 6 | 3 | 34 | 6 |
| Paraffin wax | 2 | – | 4 | – | – |
| **Water-Insoluble Plasticizers** | | | | | |
| Hydrogenated vegetable oils | 4 | 2 | 8 | 5 | – |
| Fats | – | 1 | – | – | 3 |
| Unmodified oils | – | | 2 | – | – |
| **Antioxidant** | | | | | |
| BHT | $\leq 1$ | $\leq 1$ | $\leq 1$ | $\leq 1$ | $\leq 1$ |
| | 100.0 | 100.0 | 100.0 | 100.0 | 100.0 |

EXAMPLE II

Chewing gum compositions of the present invention (e.g. gum compositions A-J of Table II, below) are prepared from the bases of this invention (e.g. the bases of Example I) by adding a gum base of the invention, as is, or premelted or softened in a heating keetle or oven to approximately 80°C, to a stainless steel sigma blade mixer. The gum base is spread in the mixer and typically the pharmaceutical, softener, bulking agent and flavorings are added in a sequential manner until a homogeneous blend of ingredients is achieved. At this point, the gum mass will have a dough-like consistency and attained a temperature of 40°-50°C. The procedure itself is well known to those of ordinary skill in the art. The gum mass is then rolled, extruded, sized or pressed into a desired gum piece of specific weight and dimension.

Specific gum formulations according to this invention are provided in Tables II-V below.

## TABLE II

| Gum Base A from Table I | 60% |
|---|---|
| Sorbitol Powder (100 mesh) | 30% |
| Glycerol Monostearate | 5% |
| Lecithin | 1% |
| Hydrogenated Cotton Seed Oil | 2% |
| Peppermint Oil | 1% |
| | 100% |

## TABLE III

| Gum Base C from Table I | 80% |
|---|---|
| Sorbitol Powder | 10% |
| Xylitol Powder | 5% |
| Glycerol Triacetate | 3.5% |
| Spearmint Oil | 1.0% |
| Caffeine | 0.5% |
| | 100% |

## TABLE IV

| Gum Base D from Table I | 90% |
|---|---|
| Mannitol Powder (100 mesh) | 5% |
| Acetylated Monoglyceride | 1.5% |
| Lecithin | 1% |
| Glycerol Monostearate | 1% |
| Spray-Dried Fruit Flavor | 0.4% |
| ASPARTAME | 0.1% |
| Sodium Chloride | 0.5% |
| Potassium Chloride | 0.5% |
| | 100% |

EP 0 472 428 A2

## TABLE V

| | |
|---|---|
| Gum Base E from Table I | 70% |
| Sucrose | 20% |
| Distilled Monoglyceride | 5% |
| Lecithin | 2% |
| Lemon Flavor Oil | 1.5% |
| Ascorbic Acid | 1.5% |
| | 100% |

## Claims

1. A gum composition, comprising:
   from 30 to 90 percent of a gum base having a softening point between 70°C and 100°C;
   from 0.1 to 20 percent of a water-insoluble plasticizer;
   from 0 to 65 percent of a solid bulk sweetener; and
   less than 0.5 percent water; and said gum being substantially free of humectant.

2. A gum composition as claimed in Claim 1 which further includes a physiologically effective amount of a pharmaceutical.

3. A gum composition as claimed in Claim 1 or Claim 2 which contains 70 to 80 percent of said gum base.

4. A gum composition as claimed in any one of claims 1 to 3 in which said gum base includes an elastomer from 5 to 75 percent by weight of said base.

5. A gum composition as claimed in claim 4 in which said elastomer is 5 to 55 percent by weight of said base.

6. A gum composition as claimed in any one of claims 1 to 5 in which the base includes a filler in an amount less than 60 percent by weight of said base.

7. A gum composition as claimed in claim 6 in which said filler comprises from 20 to 50 percent by weight of said base.

8. A gum composition as claimed in any one of claims 1 to 7 which contains 15 to 25 percent of said solid bulk sweetener.

9. A gum composition as claimed in ony one of claims 1 to 8 in which the bulk sweetener is selected from sucrose, dextrose, sorbitol, maltitol, xylitol, mannitol, palatinit, and polydextrose.

10. A gum composition as claimed in any one of claims 1 to 9 which contains 0.5 to 10 percent of said water-insoluble plasticizer.

11. A gum composition as claimed in any one of claims 1 to 10 which further includes a non-nutritive sweetener.

12. A gum composition as claimed in any one of claims 1 to 11 which further includes less than 3 percent of a flavoring.

13. A gum composition as claimed in claim 12 in which the flavoring is oil soluble.

6

**14.** A gum composition as claimed in claim 12 or claim 13 in which the flavoring is encapsulated.

**15.** A gum composition as claimed in any one of claims 12 to 14 in which the flavoring includes a food acid.

**16.** A gum composition as claimed in any one of claims 12 to 15 in which the flavoring includes a buffering agent.

**17.** A gum composition as claimed in any one of claims 1 to 17 in which the gum composition is coated.

**18.** A method of making chewing gum comprising mixing together the following components;
from 30 to 90 per cent of a gum base having a softening point between 70°C and 100°C;
from 0.1 to 0.20 per cent of a water-insoluble plasticizer;
from 0 to 65 per cent of a solid bulk sweetener;
and less than 0.5 per cent water; said components being substantially free of humectant.

**19.** A method as claimed in claim 18 in which a physiologically effective amount of a pharmaceutical is mixed together with the other components.